# EUROPÄISCHE PATENTSCHRIFT

(11) **EP 1 027 318 B1**
(45) Veröffentlichungstag und Bekanntmachung des Hinweises auf die Patenterteilung: **16.04.2003**
(21) Anmeldenummer: 98955429.0
(22) Anmeldetag: 12.10.1998
(51) Int. Cl.: C07C 47/04, C07C 47/058, C08G 2/08, C07D 323/06

(54) **VERFAHREN ZUR REINIGUNG VON FORMALDEHYD**
METHOD FOR PURIFICATION OF FORMALDEHYDE
PROCEDE PERMETTANT DE PURIFIER LE FORMALDEHYDE

(30) Priorität: 31.10.1997 DE 19748380
(43) Veröffentlichungstag der Anmeldung: 16.08.2000
(73) Patentinhaber: Ticona GmbH, 65451 Kelsterbach (DE)
(72) Erfinder: SIEVERS, Werner, D-65929 Frankfurt (DE); SCHWEERS, Elke, D-69812 Bad Soden (DE); MEISTER, Christine, D-65843 Sulzbach (DE)
(86) Internationale Anmeldenummer: EP9806440
(87) Internationale Veröffentlichungsnummer: WO99023054

(56) Entgegenhaltungen:
- GB-A- 987 457
- US-A- 4 203 735

## Beschreibung

Die Erfindung betrifft ein Verfahren zum Reinigen von Formaldehyd, und zwar insbesondere zum kontinuierlichen Reinigen von Formaldehyd, die Verwendung von derart gereinigtem Formaldehyd zur Herstellung von Kunststoffen, insbesondere von Polyacetalen und Polyoxymethylenen sowie Kunststoffe, die unter Verwendung des so gereinigten Formaldehyds hergestellt worden sind.

Formaldehyd stellt für die chemische Synthese vieler Produkte wie Kunststoffe, Düngemittel, Farbstoffe sowie bei der Spiegelherstellung und in der Textilveredelung ein wichtiges Ausgangsprodukt dar. Es wird daher in großen Mengen industriell dargestellt. Dementsprechend ist eine Vielzahl von verschiedenen Verfahren zur Herstellung von Formaldehyd bekannt, wobei die Darstellung aus Methanol die größte Bedeutung aufweist. Dabei wird mittels einer oxidativen Dehydrierung, beispielsweise an einem oxidierten Kupferdraht Formaldehyd durch Oxidation von Methanol mit Luft dargestellt. Bei dieser Synthese werden üblicherweise 30-40%ige, wäßrige Lösungen von Formaldehyd erhalten, die aus dem Herstellungsverfahren bereits das zur Stabilisierung notwendige Methanol enthalten.

In der deutschen Patentanmeldung 197 22 774.0 und der dazu korrespondierenden internationalen Patentanmeldung PCT/EP98/03084 ist die Herstellung von Formaldehyd durch direkte Dehydrierung von Methanol bei einer Temperatur von 300 bis 1000°C mittels eines Katalysators beschrieben. Dabei wird ein Kreisgasstrom, der vorwiegend aus Wasserstoff und Kohlenmonoxid besteht, durch den Reaktor geführt. Auf diese Weise entsteht ein Gemisch, das Formaldehyd, Wasserstoff, Kohlenmonoxid sowie Nebenprodukte wie Wasser, Methanol, Methylformiat, Methylal, Kohlendioxid und Methan mit Anteilen <5 Gew.-% enthält.

Alle diese Verfahren zur Herstellung von Formaldehyd weisen jedoch den Nachteil auf, daß mit ihnen Formaldehyd nicht als reiner Stoff, sondern als Gemisch mit unerwünschten Nebenprodukten und nicht umgesetzten Ausgangsstoffen erhalten wird. Für eine Mehrzahl industrieller Synthesen, wie der Herstellung technischer Kunststoffe, wird jedoch Formaldehyd in hoch reinem Zustand benötigt, da die Qualität des damit erzeugten Produktes neben den gewählten Reaktionsbedingungen auch von der Reinheit des gasförmigen Formaldehyds abhängt.

Es sind daher bereits eine Vielzahl von Verfahren entwickelt worden, um Formaldehyd aus einer wäßrigen Lösung abzutrennen und damit hochwertige Produkte herzustellen. Dabei wird Formaldehyd üblicherweise aus einer wäßrigen Lösung abgezogen und anschließend in gasförmigem Zustand weiter gereinigt. Die grundlegenden verfahrenstechnischen und stofflichen Eigenschaften von Formaldehyd sind in J.F. Walker, "Formaldehyde", Reinhold Publishing Corporation Chapman and Hall, Ltd., London, beschrieben.

Darüber hinaus ist in der DE-A 10 90 191 ein Verfahren zur kontinuierlichen Reinigung von gasförmigem Formaldehyd beschrieben, bei dem der Formaldehyd mit einer strömenden Oberfläche einer kalten, flüssigen Formaldehyd-Halbacetal-Lösung in Kontakt gebracht wird, die ihrerseits durch Umsetzung von Formaldehyd mit einem primären und sekundären Alkohol gebildet wurde. Dabei werden 60-98% der flüssigen Verunreinigungen aus dem Formaldehydgas von der kalten flüssigen Lösung absorbiert. Eine Entfernung von normalerweise gasförmigen Stoffen, wie N₂, O₂, CO₂ und CO ist jedoch, wie ausdrücklich betont wird, mit diesem Verfahren nicht möglich.

Die US-A 2,848,500 beschreibt ein Verfahren, wonach ein Formaldehyd-Wasser-Gemisch mit einem Alkohol, dessen Siedepunkt über 95°C liegt, zum Hemiformal umgesetzt wird, das anfallende Gemisch im Vakuum zu einer Hemiformallösung von erheblich geringerem Wassergehalt dehydratisiert und dann pyrolysiert wird, wobei der Formaldehyd aus dem Hemiformal freigesetzt wird, worauf durch Teilkondensation der Pyrolyseprodukte der Alkohol als Flüssigkeit und Formaldehyd als Dampf gewonnen wird. Bei diesem Verfahren wird jedoch Formaldehyd zusammen mit Wasser entfernt, wodurch die Ausbeute beträchtlich verringert wird. Daher sind weitere Verfahrensstufen erforderlich, um den aus dem Gemisch entfernten Formaldehyd wiederzugewinnen bzw. vom Wasser abzutrennen. Aus diesem Grund lehrt die DE-A 16 18 772 dem Gemisch vor dem Entwässern mindestens ein Alkalisalz eirier organischen Säure zuzusetzen.

In der US-A 3,510,525 ist ein Verfahren zur Herstellung von gasförmigem Formaldehyd beschrieben, wonach eine gasförmige Mischung aus Luft und wäßrigem Formaldehyd im Gegenstrom mit einer zirkulierenden trockenen Mischung aus Formaldehyd und mehrwertigem Alkohol zum Hemiacetal umgesetzt wird. Die verbleibende gasförmige Mischung, die nicht umgesetztes Formaldehyd enthält, wird in einem zweiten Schritt mit einer wäßrigen Formaldehyd-Alkohol-Mischung bei einer niedrigeren Temperatur umgesetzt, und schließlich wird in einem dritten Schritt der Rest des noch vorliegenden gasförmigen Formaldehyds in Wasser gelöst. Die Hemiformallösung des ersten Schritts wird dann gegebenenfalls nach einer vorgeschalteten Trocknung mittels fester Trockenmittel oder mittels trockener Luft oder einer anderen Trocknungsmethode der Pyrolyse zugeführt.

In der DE-A 41 37 846 wird ein Verfahren zur Herstellung von weitgehend wasserfreiem und von Verunreinigungen befreitem Formaldehyd aus wäßrigen Formaldehydlösungen beschrieben, wobei die wäßrige Lösung mit einem Alkohol extrahiert, die so erhaltene alkoholische Halbacetallösung vom Wasser befreit und danach in einer nachfolgenden Lösungsmittelwechselkolonne in Gegenwart eines Lösungsmittels mit einem tieferen Siedepunkt als der bei der Extraktion verwendete Alkohol thermisch gespalten und als Kopfprodukt Formaldehyd im Gemisch mit dem Lösungsmittel abgezogen wird. In der DE-A 12 49 846 wird die thermische Zersetzung von weitgehend reinen Halbformalen in Kolonnen mit beheizten Böden bei Temperaturen zwischen 20 und 250°C und Verweilzeiten von 1 bis 60 Min. beschrieben, wobei das Hemiformal durch spiralförmige oder als Umlenkbleche ausgebildete Einbauten entlang den Heizflächen zwangsgeführt wird.

Aus der GB-A-987,457 ist ein Verfahren zur Reinigung von Formaldehyd bekannt, bei dem hochsiedende Alkohole mit einem niedrigen Dampfdruck eingesetzt werden. Gemäß dieser Schritt wird eine Pyrolyseeinheit so betrieben, dass im wesentlichen nur Formaldehyd verdampft, während Alkohol in der Pyrolyseeinheit zurückbleibt.

Alle zuvor beschriebenen Verfahren weisen jedoch Nachteile bezüglich der Reinheit des erhaltenen Formaldehyds oder bei der Ausbeute auf, oder sie sind nicht kontinuierlich durchführbar. Der Erfindung lag daher die Aufgabe zugrunde, ein Reinigungsverfahren für Formaldehyd bereitzustellen, welches die zuvor geschilderten Nachteile nicht aufweist und mit dem auch im kontinuierlichen Verfahren Formaldehyd unter Bildung von Halbacetalen auf einfache Weise ohne wesentlichen Ausbeuteverlust im hoch reinen Zustand erhältlich ist.

Dies wird erfindungsgemäß dadurch erreicht, daß man den Formaldehyd in einer Absorptionseinheit mit einem Alkohol wäscht und den Formaldehyd dabei nahezu vollständig im Alkohol löst bzw. zum Hemiformal umsetzt und gleichzeitig in einem ersten Reinigungsschritt gasförmige Verunreinigungen, die nicht von der Waschlösung absorbiert werden, abtrennt. Danach wird die Formaldehyd-Alkohol-Hemiformal-Lösung in eine Desorptionseinheit geleitet, worin die in der Absorptionseinheit von der Alkohol-Hemiformal-Lösung absorbierten restlichen Verunreinigungen in einen gasförmigen Zustand überführt und so vom Hemiformal abgetrennt werden. Das auf diese Weise gereinigte Hemiformal wird dann in einer Pyrolyseeinheit zum Alkonol und Formaldehyd gespalten, und durch Austragen von Alkohol und Formaldehyd und Kondensation des Alkohos wird der Formaldehyd zurückgewonnen. Im erfindungsgemäßen Verfahren wird vorzugsweise Formaldehyd im gasförmigen Zustand gereinigt, wie er direkt bei der Synthese anfällt. Es ist jedoch auch möglich, Formaldehyd in anderen Formen, z. B. als Formalin, einzusetzen, vorzugsweise wird er jedoch auch hier zuerst in den gasförmigen Zustand überführt.

Als Absorptionseinheit werden vorzugsweise Absorptionskolonnen, insbesondere Oberflächenrektifikatoren, wie gepackte Kolonnen oder Bodenkolonnen verwendet, wobei Bodenkolonnen ein großes Rückhaltevermögen und gepackte Kolonnen eine große Oberfläche aufweisen. Besonders bevorzugte Kolonnen sind Glockenbodenkolonnen, Sprühkolonnen, Rieselkolonnen und Füllkorperkolonnen. Um eine möglichst hohe Absorption von Formaldehyd zu erreichen, werden vorzugsweise Kolonnen mit solchen Einbauten eingesetzt, die eine möglichst große Austauschfläche bereitstellen.

Das erfindungsgemäße Verfahren wird vorzugsweise derart durchgeführt, daß der Alkohol in die Absorptionseinheit im Gegenstrom zum Formaldehyd eingeleitet wird. Es hat sich nämlich gezeigt, daß auf diese Weise die mit dem Formaldehyd eingetragenen gasförmigen Neben- und Ausgangsprodukte, insbesondere das Wasserstoff/Kohlenmonoxid-Gasgemisch des Kreisgases, vom Alkohol absorbierte Substanzen wie Methylformiat, Kohlenmonoxid sowie andere Komponenten aus der Hemiformallösung im oberen Bereich der Kolonne wieder herausstrippen. Auf diese Weise ist es möglich, sowohl die Kreisgasabtrennung, die Hemiformalbildung und eine weitere Reinigung der Hemiformallösung durch Strippen in einer einzigen Apparatur durchzuführen. Darüber hinaus kann aufgrund dieser Reinigung der Hemiformallösung auf die Desorptionseinheit bei entsprechender Prozeßführung in der Absorptionseinheit gegebenenfalls verzichtet werden.

Für das erfindungsgemäße Verfahren sind sowohl ein- als auch mehrwertige Alkohole verwendbar, sofern deren Siedepunkt, insbesondere bei Normaldruck, deutlich über demjenigen von Wasser und deutlich unter der Pyrolysetemperatur des entstehenden Hemiformais liegt. Zur Vermeidung von weiteren unerwünschten Nebenreaktionen ist es bevorzugt, Alkohole zu verwenden, die selbst keine reaktiven Gruppen wie Carbonyl-, Nitro- oder Amingruppen enthalten. Besonders bevorzugte Alkohole sind Alkohole mit 1 bis 8 C-Atomen, insbesondere 3 bis 7 C-Atomen, wobei Cyclohexanol besonders bevorzugt ist.

Zur Vermeidung von unerwünschten Nebenreaktionen wird die Formaldehydabsorption bei Temperaturen oberhalb 0°C, vorzugsweise im Bereich von 5 bis 45°C, insbesondere im Bereich von 15 bis 40°C durchgeführt, wobei Temperaturen im Bereich von 20 bis 30°C besonders bevorzugt sind. Die Absorption wird zweckmäßiger Weise bei Umgebungsdruck durchgeführt. Es ist jedoch auch möglich, die Absorption mit leicht erhöhtem Druck oder unter schwachem Vakuum durchzuführen. Für das erfindungsgemäße Verfahren hat es sich ais zweckmäßig erwiesen, die Absorption bei möglichst tiefen Temperaturen durchzuführen, wobei die untere Grenze durch die Viskosität des Alkohols bestimmt wird. Z. B. weist Cyclohexanol bei Raumtemperatur eine Viskosität von 6,8x10⁻² Ns/m² auf.

Da mit steigender Beladung von Formaldehyd, d. h. mit zunehmender Hemiformalkonzentration die alkoholische Lösung zunehmend dünnflüssiger wird, hat es sich in besonderen Fällen auch als zweckmäßig erwiesen, in die Absorptionseinheit anstatt eines reinen Alkohols eine Alkohol-Hemiformal-Lösung einzutragen, um so die Absorptionstemperatur herabzusetzen. Besonders zweckmäßig wird dabei der aus der Pyrolyse rückgewonnene Alkohol mit der in der Desorptionseinheit gereinigten Hemiformallösung versetzt, oder es wird eine unvollständig gespaltene Alkohol-Hemiformal-Lösung aus der Pyrolyseeinheit rückgeführt.

Beim erfindungsgemäßen Verfahren wird die Hemiformalbildung, bzw. die Reaktionstemperatur, über den Alkoholvolumenstrom, in zweckmäßiger Weise über den Alkoholeintrag und/oder den Hemiformalaustrag, gesteuert. Dabei hat es sich als vorteilhaft erwiesen, daß die Bildungsreaktion derart geregelt wird, daß Formaldehyd im Austragsbereich in einer Menge von 50 bis 500 g/l, vorzugsweise 150 bis 350 g/l und insbesondere zwischen 200 und 300 g/l in der Lösung als Hemiformal vorliegt. Bei dem erfindungsgemäßen Verfahren werden weder das Kohlenmonoxid noch Wasserstoff absorbiert, so daß es möglich ist, das gesamte Kreisgas ohne weiteres vom Formaldehyd im ersten Reaktionsschritt abzutrennen. Obwohl hierbei nahezu der gesamte Formaldehyd zu Hemiformal umgesetzt wird, werden die Nebenkomponenten nur in begrenztem Umfang von der Alkohol-Hemiformal-Lösung absorbiert, so daß auch diese zum Großteil im ersten Absorptionsschritt im gasförmigen Zustand abgetrennt werden können.

Die aus der Absorptionseinheit ausgetragene und vorgereinigte Hemiformallösung wird, gemäß dem erfindungsgemäßen Verfahren, in eine Desorptionseinheit eingeleitet. In der Desorptionseinheit werden die in der Hemiformal-Alkohol-Lösung absorbierten bzw. gelösten Nebenprodukte in einen gasförmigen Zustand überführt und von der flüssigen Hemiformallösung abgetrennt. Die Desorption, d. h. die Überführung der gelösten Verunreinigungen, wird im erfindungsgemäßen Verfahren durch eine Erhöhung der Temperatur und/oder durch Anlegen eines Vakuums erreicht.

Darüber hinaus hat es sich als zweckmäßig erwiesen, die Desorption mittels eines Inertgases, vorzugsweise mittels Stickstoff, durchzuführen. Prinzipiell sind je nach Art des verwendeten Alkohols und der vorliegenden Verunreinigungen eine möglichst hohe Temperatur oder Vakuum einzusetzen. Da allerdings mit zunehmender Temperatur verstärkt unerwünschte Nebenreaktionen, beispielsweise die Bildung von Methylformiat, einsetzen, sollte die Temperatur einen bestimmten Grenzwert nicht überschreiten. Auf diese Weise werden leicht flüchtige Komponenten wie Wasser, Methanol und Methylformiat ausgetrieben, und zwar ohne wesentliche Formaldehydverluste.

Bei einer außergewöhnlich großen Wasserbeladung hat es sich als zweckmäßig erwiesen, anstatt oder zusätzlich zur Desorptionseinheit eine Vakuumdestillation durchzuführen.

Bei einer nicht zu hohen Beladung der aus der Absorptionseinheit austretenden Hemiformallösung mit Nebenkomponenten kann auf die Desorptionseinheit verzichtet werden. Die Hemiformallösung wird dann direkt der Pyrolyseeinheit zugeleitet.

Im erfindungsgemäßen Verfahren wird die Desorption im allgemeinen bei Temperaturen im Bereich von 20 bis 100°C, insbesondere von 30 bis 70°C und vorzugsweise bei 40 bis 60°C und/oder bei einem Vakuum von 1 bis 1000 mbar, insbesondere 1000 bis 50000 N/m² (10 bis 500 mbar) und vorzugsweise bei 2000 bis 10000 N/m² (20 bis 100 mbar) durchgeführt. Da das Hemiformal üblicherweise erst bei höheren Temperaturen gespalten wird und üblicherweise einen höheren Siedepunkt als der Alkohol und die Verunreinigungen aufweist, ist dies üblicherweise die höchstsiedendste Komponente der Alkohol-Hemiformal-Lösung.

Als Desorptionseinheit werden vorzugsweise Kolonnen verwendet, und zwar insbesondere gepackte Kolonnen sowie auch Bodenkolonnen. Da die Desorptionseinheit auch eine zur Vakuumdestillation geeignete Vorrichtung sein kann, haben sich sämtliche für die Destillation geeignete Kolonnen als zweckmäßig erwiesen. Besonders bevorzugt ist es, die Alkohol-Hemiformal-Lösung in den oberen Teil der Desorplionseinheit einzuleiten und im Gegenstrom hierzu das Inertgas einzutragen und zwar vorzugsweise im unteren Teil der Kolonne, so daß durch das Inertgas die leichter flüchtigen Verunreinigungen aus der Lösung gestrippt werden können. Dies bewirkt einen zusätzlichen Reinigungseffekt.

Das aus der Desorptionskolonne ausgetragene gereinigte Hemiformal wird dann in eine Pyrolyseeinheit geleitet, in der es thermisch zum Formaldehyd und zum Alkohol gespalten wird. Dabei hat es sich als zweckmäßig erwiesen, die Pyrolysebedingungen derart auszuwählen, daß die Alkohol-Hemiformal-Lösung in der Pyrolyseeinheit möglichst kurz verweilt, um unerwünschte Nebenreaktionen, d. h. die Neubildung von Verunreinigungen, zu vermeiden.

Bevorzugte Pyrolyseeinheiten sind Verdampfer, wobei es bevorzugt ist, Dünnschichtverdampfer zu verwenden, und zwar vorzugsweise solche, die mit einer möglichst hohen Drehzahl betrieben werden können.

Bei der Verwendung von Cyclohexanol als Alkohol hat es sich als-zweckmäßig erwiesen, die Pyrolyse in einem Temperaturbereich von 100 bis 200°C, vorzugsweise von 120 bis 180°C und insbesondere bei einer Temperatur von 150 bis 175°C und/oder bei Drucken von 3000 bis 150000 N/m² (30 bis 1500 mbar), insbesondere von 5000 bis 13000 N/m² (50 bis 1300 mbar) durchzuführen. Das aus der Pyrolyse ausgetragene Gasgemisch enthält Formaldehyd und den Alkohol.

Gegebenenfalls vorliegende schwer flüchtige Verunreinigungen und Nebenprodukte werden als Flüssigkeit oder Feststoff im Pyrolysator abgetrennt. Im ausgetragenen Gasgemisch wird dann der höhersiedende Alkohol in einem Kondensator gegebenenfalls zusammen mit Hemiformalen rückkondensiert und kann so gegebenenfalls nach weiteren Reinigungsschritten für weitere Absorptions- und Desorptionsvorgânge im erfindungsgemäßen Verfahren rückgeführt werden. Der nicht kondensierte Formaldehyd wird als reiner Gasstrom erhalten, der frei von Kohlenmonoxid und Wasserstoff und im wesentlichen frei von Methanol ist. Mit dem erfindungsgemäßen Verfahren sind ohne weiteres Reinheitsgrade zu erreichen, bei denen der Anteil von Methanol kleiner 100 ppm, insbesondere kleiner 50 ppm und vorzugsweise kleiner 30 ppm ist.

Der nach dem erfindungsgemäßen Verfahren hergestellte Formaldehyd eignet sich für alle bekannten Einsatzgebiete, beispielsweise Korrosionsschutz, Spiegelherstellung, elektrochemische Beschichtungen, zur Desinfektion und als Konservierungsmittel, weiter als Zwischenprodukt zur Herstellung von Polymeren wie Polyacetalen, insbesondere Polyoxymethylen, Phenolharzen, Polyurethanen, Melaminharzen, Aminoplasten, Phenoplasten und Caseinkunststoffen, sowie von 1,4-Butanolen, Trimethylolpropan, Neopentylglykol, Pentaerythrit, alkoholischen Formaldehydlösungen, Farbstoffen, wie Fuchsin und Acridin, und Düngemitteln sowie zur Behandlung von Saatgut. Vorzugsweise wird er zur Herstellung der genannten Kunststoffe verwendet.

Da Formaldehyd nach dem erfindungsgemäßen Verfahren üblicherweise mit geringem Wassergehalt hergestellt wird, eignet sich so hergestellter Formaldehyd insbesondere für die Polymerisation zu Polyoxymethylen Polyacetalen und Trioxan, da hier wasserfreier Formaldehyd einzusetzen ist, mit den hergestellten Formaldehyd hergerstellte

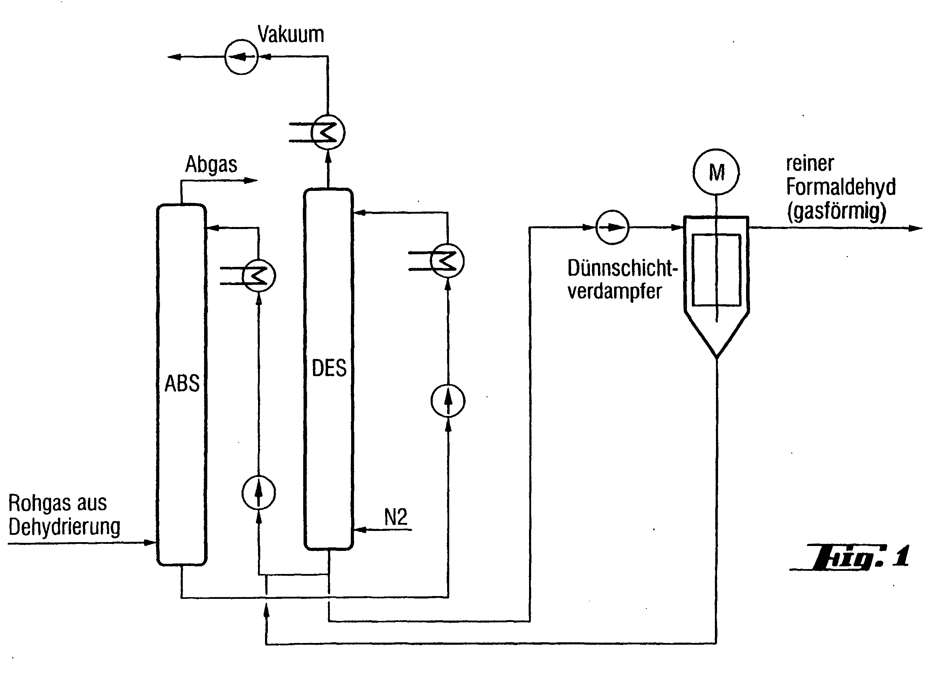

Die Erfindung soll anhand der beiliegenden Figur 1, die eine Vorrichtung zur Durchführung des erfindungsgemäßEn Verfahrens zeigt, näher erläutert werden.

Wie Figur 1 zu entnehmen ist, weist die als Kolonne bzw. Säule ausgebildete Absorptionseinheit (1) in ihrem unteren Teil einen Einlaß für den Eintrag des aus der Dehydrierung kommenden Rohgases (2) sowie einen stromabwärts liegenden Auslaß für die gebildete Alkohol-Hemiformal-Lösung auf. Im oberen Bereich enthält die Säule eine Eintragsöffnung für den Alkohol sowie eine weiter stromaufwärts liegende Öffnung für den Austritt der Abgase und gasförmigen Verunreinigungen (3). Die aus der Absorptionseinheit (1) kommende vorgereinigte Alkohol-Hemiformal-Lösung wird in eine Desorptionseinheit (4) geleitet, die ebenfalls als Kolonne oder Säule ausgebildet ist. Die Desorptionseinheit (4) weist im unteren Teil eine verfahrensstromabwärts liegende Austrittsöffnung für die gereinigte. d. h. desorbierte Alkohol-Hemiformal-Lösung auf sowie eine Öffnung zum Eintrag des Inertgases (N₂), das im Gegenstrom zur Alkohol-Hemiformal-Lösung durch die Desorptionseinheit geleitet wird. Am oberen Ende weist die Desorptionseinheit eine Austrittsöffnung für die flüchtigen bzw. durch das Inertgas gestrippten Verunreinigungen auf, die über einen Verdichter abgezogen werden (5).

Die aus der Desorptionseinheit ausgetragene und von Verunreinigungen gereinigte Alkohol-Hemiformal-Lösung wird dann über eine Eintragsöffnung in die Pyrolyseeinheit (6) eingetragen und dort thermisch gespalten. Der dabei entstehende Alkohol wird wiedergewonnen und gegebenenfalls unter Vermischung mit einem Teil des gereinigten Hemiformals der Desorptionseinheit (4) zur Wiederverwendung in die Absorptionseinheit (1) rückgeführt. Der reine Formaldehyd Wird als gasförmiger Produktstrom (7) am Kopfende der Pyrolyseeinheit (6) entnommen.

Die Erfindung wird anhand folgender Beispiele näher erläutert:

### Beispiel 1: Hemiformalbildung in der Absorptionskolonne

An einer wie in Figur 1 dargestellten Vorrichtung, bei der als Absorptionskolonne (1) eine Bodenkolonne im Labormaßstab mit einem Durchmesser von 50 mm und einer Höhe von 1,5 m sowie 22 Glockenböden verwendet wird, wird ein Formaldehyd-Rohgas-Gemisch mit einer Eintragsgeschwindigkeit von V=147 Normliter/Std. und einer Temperatur von 80° C eingeleitet. Dabei wird die Absorptionstemperatur über den Eintrag der Waschflüssigkeit eingestellt.

Der Einfluß der Absorptionstemperatur und der Flüssigkeitsbelastung, d. h. der Umpumpmenge des Alkohols Cyclohexanol auf die Reaktion von Formaldehyd und selektiver Abtrennung von Nebenkomponenten wird anhand von fünf verschiedenen Versuchsbeispielen gezeigt. Dabei betrug die Umpumpmenge zwischen 10 l/Std. (entspricht einer Flüssigkeitsbelastung von B=5m³ pro m² und Std.) und 15 I/Std. (entspricht B=7,5 m³ pro m² und Std.), wobei die verwendete Laborbodenkolonne bei 20 I/Std. flutet. Die Eintrittstemperatur des Waschmittels wurde zwischen 0°C, 20°C und 40°C variiert, wie dies in Tabelle 1 angegeben ist.

**Tabelle 1:**

| Variation von Temperatur und Volumenstrom des Cyclohexanols | | |
|---|---|---|
| Versuch | Volumenstrom [l/h] | Temperatur [°C] |
| 1 | 10 | 40 |
| 2 | 15 | 40 |
| 3 | 15 | 20 |
| 4 | 15 | 0 |
| 5 | 10 | 20 |

In der Tabelle 2 sind die Ein- und Ausgangskonzentrationen sowie die Molenströme angegeben. Die Gasleerrohrgeschwindigkeit betrug dabei ca. 0,02 m/sec und das Molstromverhältnis von Flüssigkeit zu Gasstrom betrug UG = 95 Mol/Std. : 6,6 Mol/Std = 14,4 Mol/Mol (Dichte von Cyclohexanol (CHol) bei 20°C: 952 kg/m³, molare Masse: 100,2 kg/kmol).

**Tabelle 2:**

| Versuchsergebnisse (Konzentrationen im Gas) | | | | | | |
|---|---|---|---|---|---|---|
| T=40°C, V(Chol)=10 l/h | | | | | | |
| Versuch 6 | | gesamt | Formaldehyd | Wasser | Methanol | Methylformiat |
| Rohgas- | ppm | | 270000 | 2000 | 300 | 1700 |
| zufuhr (2) | | | | | | |
| | Mol/h | 6,6 | 1,782 | 0,0122 | 0,00100 | 0,0101 |
| Abgas (3) | ppm | | 250000 | 1000 | 100 | 1300 |
| | Mol/h | 4,9 | 0,1225 | 0,09490 | 0,000490 | 0,00637 |
| % im Abgas | | | 6,9 | 37,1 | 24,8 | 63,1 |

| T=40°C, V(CHol) = 15 l/h | | | | | | |
|---|---|---|---|---|---|---|
| Versuch 7 | | gesamt | Formaldehyd | Wasser | Methanol | Methylformiat |
| Rohgas- | ppm | | 270000 | 1000 | 150 | 1300 |
| zufuhr (2) | | | | | | |
| | mol/h | 6,6 | 1,782 | 0,0066 | 0,00099 | 0,00883 |
| Abgas (3) | ppm | | 2000 | 600 | 90 | 600 |
| | mol/h | 4,9 | 0,098 | 0,00294 | 0,000441 | 0,00294 |
| % im Abgas | | | 5,5 | 44,5 | 44,6 | 34,3 |

| T=20°C, V(CHol) = 15 l/h | | | | | | |
|---|---|---|---|---|---|---|
| Versuch 8 | | gesamt | Formaldehyd | Wasser | Methanol | Methylformiat |
| Rohgas- | ppm | | 270000 | 1000 | 100 | 300 |
| zufuhr (2) | | | | | | |
| | Mol/h | 6,6 | 1,782 | 0,0066 | 0,00066 | 0,00198 |
| Abgas (3) | Ppm | | 23000 | 300 | 70 | 220 |
| | Mol/h | 4,9 | 0,1127 | 0,00147 | 0,000343 | 0,001078 |
| % im Abgas | | | 6,3 | 22,3 | 52,0 | 54,4 |

| T = 0°C, V(CHol) = 15 l/h | | | | | | |
|---|---|---|---|---|---|---|
| Versuch 9 | | gesamt | Formaldehyd | Wasser | Methanol | Methylformiat |
| Rohgas- | Ppm | | 270000 | 1000 | 100 | 300 |
| zufuhr (2) | | | | | | |
| | mol/h | 6,6 | 1,782 | 0,0066 | 0,00066 | 0,00198 |
| Abgas (3) | ppm | | 23000 | 250 | 50 | 200 |
| | mol/h | 4,9 | 0,1127 | 0,001225 | 0,000245 | 0,00098 |
| % im | | | 6,3 | 18,6 | 37,1 | 49,5 |
| Abgas | | | | | | |

Aus Tabelle 2 ist zu entnehmen, daß die Umsetzung von Formaldehyd nur in geringem Maße von der Temperatur abhängig ist.

In Tabelle 3 ist die Abtrennung der Verunreinigungen bezogen auf Formaldehyd bei der verwendeten Laborkolonne angegeben.

**Tabelle 3:**

| Nebenproduktabtrennung und -selektivität in der Absorptionskolonne (1), in Cyclohexanol nach 5,5 Std. Versuchsdauer (Versuch 6) | | | |
|---|---|---|---|
| | im Rohgas | im Cyclohexanol | prozentuale Abreicherung |
| Methanol zu Formaldehyd | 0,11 % | 0.08 % | 0,02 |
| Wasser zu Formaldehyd | 0,74 % | 0,5 % | 0,15 |
| Methylformiat zu Formaldehyd | 0.22 % | 0,17 % | 0,03 |

Hierbei ist zu beobachten, daß beim erfindungsgemäßen Verfahren bereits in der Absorptionseinheit (1) überraschenderweise eine Aufreinigung stattfindet, was dazu führt, daß auf die Desorptionseinheit (4) bei geeigneter Prozeßführung in der Absorptionseinheit (1) gegebenenfalls verzichtet werden kann.

### Beispiel 2: Abtrennung der Verunreinigungen in den Desorptionskolonnen

In einer Desorptionskolonne (4) (DN-50-Laborkolonne mit 2m Länge und je mit einem Meter Packung Sulzer CY und Sulzer BX beschickt) gemäß der Figur 1 wurden die aus der Absorptionseinheit (1) von Beispiel 2 erhaltenen Lösungen eingetragen, wobei die Nebenprodukte zusätzlich im Vakuum mit Stickstoff gestrippt wurden. Die Ergebnisse sind in Tabelle 4 angegeben, wobei das Vakuum variiert wurde und die Temperatur über die Waschlösung eingestellt wurde.

**Tabelle 4:**

| Gaskonzentration im Desorptionsstrom | | | | | | |
|---|---|---|---|---|---|---|
| (Trägergas: Stickstoff mit 20 liter/Std) | | | | | | |
| Versuch | Temp. [°C] | Druck [N/m²] ([mbar]) | Formaldehyd [ppm] | Wasser [ppm] | Methanol [ppm] | Methylformiat |
| | | | | | | [ppm] |
| 10 | 26 | 4000 (40) | 380 | 8000 | 220 | 1800 |
| 11 | 26 | 4000 (40) | 500 | 15800 | 100 | 500 |
| 12 | 26 | 1000 (10) | 500 | 1500 | 70 | 300 |
| 13 | 26 | 1000 (10) | 380 | 1000 | 120 | 1000 |
| 14 | 01 | 2000 (20) | 140 | 2700 | 250 | 4000 |

In Tabelle 5 sind die molaren Verhältnisse von Wasser/Methanol/Methylformiat zu Formaldehyd sowie der Aufkonzentrieruncsfaktor (bezogen auf das Rohgemisch) angegeben.

**Tabelle 5a:**

| Verhältnis Wasser zu Formaldehyd | | | | | |
|---|---|---|---|---|---|
| Versuch | Desorptions temp. [°C] | Desorptions druck [N/m²] ([mbar]) | Wasser zu Formaldehyd im Rohgas | Wasser zu Formaldehyd im Desorptions-gas | Faktor Aufkonzentrierung |
| 15 | 40 | 8000 (80) | 1:270 | 1:4 | ca. 70 |
| 16 | 40 | 50000 (500) | 1:270 | 1:9 | ca. 30 |
| 17 | 10 | 50000 (500) | 1:270 | 1:11 | ca. 25 |
| 18 | 10 | 8000 (80) | 1:270 | 1:7 | ca. 40 |
| 19 | 20 | 4000 (40) | 1:270 | 1:3 | ca. 90 |

**Tabelle 5b:**

| Verhältnis Methanol zu Formaldehyd | | | | | |
|---|---|---|---|---|---|
| Versuch | Desorptions temp, [°C] | Desorptions druck [N/m²] ([mbar]) | Methanol zu Formaldehyd im Rohgas | Methanol zu Formaldehyd im Desorptionsgas | Faktor Aufkonzentrierung |
| 15 | | 38000 (380) | 1:1800 | 1:36 | ca. 50 |
| 16 | 26 | 50000 (500) | 1:1800 | 1:70 | ca. 30 |
| 17 | 26 | 50000 (500) | 1:1800 | 1:79 | ca. 20 |
| 18 | 26 | 38000 (380) | 1:1800 | 1:58 | ca. 30 |
| 19 | 01 | 14000 (140) | 1:2250 | 1:40 | ca. 60 |

**Tabelle 5c:**

| Verhältnis Methylformiat zu Formaldehyd | | | | | |
|---|---|---|---|---|---|
| Versuch | Desorptions temp. [°C] | Desorptions druck [N/m²] ([mbar]) | Methylformiat zu Formaldehyd im Rohgas | Methylformiat zu Formaldehyd im Desorptions-gas | Faktor Aufkonzentrierung |
| 15 | 40 | 38000 (380) | 1:450 | 1:4,4 | ca. 100 |
| 16 | 406 | 50000 (500) | 1:450 | 1:14 | ca. 30 |
| 17 | 10 | 50000 (500) | 1:450 | 1:18 | ca. 25 |
| 18 | 10 | 8000 (80) | 1:450 | 1:7 | ca. 60 |
| 19 | 20 | 4000 (40) | 1:900 | 1:2,5 | ca. 300 |

Vorteilhaft für eine nachhaltige Desorption der Nebenprodukte ist eine möglichst hohe Temperatur bei möglichst tiefem Vakuum. Die Obergrenze der Temperatur wird durch die Verdampfung des Waschmittels (Cyclohexanol) und zum anderen durch Abbaureaktionen, die ihrerseits Nebenprodukte erzeugen, festgelegt. Vorteilhaft sind 20 bis 80°C bei einem Vakuum von unter 10000 N/m² (100 mbar), vorzugsweise zwischen 1000 und 10000 N/m² (10 und 100 mbar).

Das in diesem Versuchsaufbau von Nebenprodukten abgereinigte Cyclohexanol wurde in einem Dünnschichtverdampfer (6) bei Temperaturen zwischen 120 und 180°C, und einem Druck im Bereich vor 5000 und 130000 N/m² (60 bis 1300 mbar) pyrolysiert.

## Patentansprüche

1. Verfahren zur Reinigung von Formaldehyd, bei dem Formaldehyd mit Alkohol zum Hemiformal umgesetzt und das so gebildete Hemiformal von unerwünschten Ausgangs- und Synthese-Nebenprodukten und/oder Verunreinigungen befreit und das so gereinigte Hemiformal zum Formaldehyd pyrolysiert wird, wobei man in einer Absorptionseinheit den Formaldehyd mit einem Alkohol wäscht und dabei diesen nahezu vollständig zum Hemiformal umsetzt und gleichzeitig in einem ersten Reinigungsschritt von in der Waschlösung nicht absorbierten gasförmigen Verunreinigungen abtrennt, in einer Desorptionseinheit die restlichen Verunreinigungen in einen gasförmigen Zustand überführt und von dem flüssigen Hemiformal abtrennt, das so gereinigte Hemiformal in einer Pyrolyseeinheit zum Alkohol und Formaldehyd spaltet, aus der Pyrolyseeinheit ein Gasgemisch enthaltend Alkohol und Formaldehyd austrägt und den höhersiedenden Alkohol in einem Kondensator gegebenenfalls zusammen mit Hemiformalen rückkondensiert und den Formaldehyd als reinen Gasstrom rückgewinnt.

2. Verfahren nach Anspruch 1, **dadurch gekennzeichnet, dass** man gasförmigen Formaldehyd verwendet.

3. Verfahren nach einem der vorhergehenden Ansprüche, **dadurch gekennzeichnet, dass** der Formaldehyd und der Alkohol zueinander im Gegenstrom in die Absorptionskolonne eingeleitet werden.

4. Verfahren nach einem der vorhergehenden Ansprüche, **dadurch gekennzeichnet, dass** man in der Absorptionskolonne eine Temperatur im Bereich von 15 bis 40°C aufrechterhält.

5. Verfahren nach Anspruch 1, **dadurch gekennzeichnet, dass** man als Alkohol Cyclohexanol verwendet.

6. Verfahren nach einem der vorhergehenden Ansprüche, **dadurch gekennzeichnet, dass** man in der Desorptionseinheit die Verunreinigungen unter Erwärmung und/oder unter Vakuum abtrennt und /oder mit einem Hilfsgas herausstrippt.

7. Verfahren nach einem der vorhergehenden Ansprüche, **dadurch gekennzeichnet, dass** man als Pyrolyseeinheit einen Verdampfer verwendet.

8. Verfahren nach einem der vorhergehenden Ansprüche, **dadurch gekennzeichnet, dass** man die Bildung von Hemiformal in der Absorptionskolonne über die Waschmittelmenge (Alkohol-Volumenstrom) steuert.

9. Verfahren nach einem der vorhergehenden Ansprüche, **dadurch gekennzeichnet, dass** Desorptionstemperaturen und Desorptionsdrücke verwendet werden, die oberhalb der Verdunstungsbedingungen von Wasser, jedoch unterhalb der Pyrolysebedingungen des Hemiformals liegen.

10. Verfahren nach einem der vorhergehenden Ansprüche, **dadurch gekennzeichnet, dass** man in der Desorptionskolonne als Inertgas Stickstoff verwendet.

11. Verfahren nach einem der vorhergehenden Ansprüche, **dadurch gekennzeichnet, dass** die Desorptionskolonne entfällt und das in der Absorptionseinheit gebildete Hemiformal direkt der Pyrolyseeinheit zugeführt wird.

12. Verfahren nach einem der vorhergehenden Ansprüche, **dadurch gekennzeichnet, dass** man Formaldehyd verwendet, der durch katalytische Dehydrierung von Methanol erhalten wurde.

## Claims

1. A process for purifying formaldehyde in which formaldehyde is reacted with alcohol to form the hemiformal and the hemiformal thus formed is freed from unwanted feedstocks and synthesis by-products and/or impurities and the hemiformal thus purified is pyrolyzed to form formaldehyde, which comprises scrubbing the formaldehyde in an absorption unit with an alcohol and in this case reacting this virtually completely to give the hemiformal and, simultaneously, separating it off, in a first separation step, from gaseous impurities not absorbed in the scrubbing solution, converting the residual impurities into a gaseous state in a desorption unit and separating them off from the liquid hemiformal, cleaving the hemiformal thus purified to the alcohol and formaldehyde in a pyrolysis unit, discharging a gas mixture comprising alcohol and formaldehyde from the pyrolysis unit and recondensing the higher-boiling alcohol in a condenser, if appropriate together with hemiformals, and recovering the formaldehyde as a pure gas stream.

2. The process as claimed in claim 1, wherein gaseous formaldehyde is used.

3. The process as claimed in one of the preceding claims, wherein the formaldehyde and the alcohol are introduced into the absorption column in countercurrent to one another.

4. The process as claimed in one of the preceding claims, wherein a temperature in the range from 15 to 40°C is maintained in the absorption column.

5. The process as claimed in claim 1, wherein the alcohol used is cyclohexanol.

6. The process as claimed in one of the preceding claims, wherein, in the desorption unit, the impurities are separated off with heating and/or under reduced pressure and/or are stripped off using an auxiliary gas.

7. The process as claimed in one of the preceding claims, wherein the pyrolysis unit used is an evaporator.

8. The process as claimed in one of the preceding claims, wherein the formation of hemiformal in the absorption column is controlled via the scrubbing medium rate (volumetric flow rate of alcohol).

9. The process as claimed in one of the preceding claims, wherein desorption temperatures and desorption pressures are employed which are above the evaporation conditions of water, but are below the pyrolysis conditions of the hemiformal.

10. The process as claimed in one of the preceding claims, wherein the inert gas used in the desorption column is nitrogen.

11. The process as claimed in one of the preceding claims, wherein the desorption column is omitted and the hemiformal formed in the absorption unit is fed directly to the pyrolysis unit.

12. The process as claimed in one of the preceding claims, wherein use is made of formaldehyde which was obtained by catalytic dehydrogenation of methanol.

## Revendications

1. Procédé de purification du formaldéhyde, lors duquel le formaldéhyde réagit avec de l'alcool pour former un hémiformal et l'hémiformal ainsi formé est débarrassé de produits secondaires d'origine et de synthèse et/ou d'impuretés non souhaités et l'hémiformal ainsi purifié subit une pyrolyse pour former le formaldéhyde, le formaldéhyde étant lavé à l'aide d'un alcool dans une unité d'absorption et réagissant en l'occurrence presque complètement pour former l'hémiformal et étant séparé simultanément, dans une première étape de purification, des impuretés gazeuses non absorbées dans la solution de lavage, les impuretés restantes étant converties à l'état gazeux dans une unité de désorption et étant séparées de l'hémiformal liquide, l'hémiformal ainsi purifié étant clivé dans une unité de pyrolyse en alcool et en formaldéhyde, un mélange gazeux contenant de l'alcool et du formaldéhyde étant évacué de l'unité de pyrolyse et l'alcool à point d'ébullition élevé étant condensé en retour dans un condenseur, le cas échéant, conjointement aux hémiformals et le formaldéhyde étant récupéré en tant que courant gazeux pur.

2. Procédé selon la revendication 1, **caractérisé en ce que** l'on utilise du formaldéhyde sous forme gazeuse.

3. Procédé selon l'une quelconque des revendications précédentes, **caractérisé en ce que** le formaldéhyde et l'alcool sont introduits dans la colonne d'absorption à contre-courant.

4. Procédé selon l'une quelconque des revendications précédentes, **caractérisé en ce que** l'on maintient, dans la colonne d'absorption, une température dans le domaine de 15 à 40°C.

5. Procédé selon la revendication 1, **caractérisé en ce que** l'on utilise, en tant qu'alcool, le cyclohexanol.

6. Procédé selon l'une quelconque des revendications précédentes, **caractérisé en ce que** l'on sépare les impuretés dans l'unité de désorption par échauffement et/ou sous vide et/ou **en ce que** l'on élimine ces dernières par stripping à l'aide d'un gaz auxiliaire.

7. Procédé selon l'une quelconque des revendications précédentes, **caractérisé en ce que** l'on utilise en tant qu'unité de pyrolyse un évaporateur.

8. Procédé selon l'une quelconque des revendications précédentes, **caractérisé en ce que** l'on contrôle la formation de l'hémiformal dans la colonne d'absorption par l'intermédiaire de la quantité d'agent de lavage (courant volumique d'alcool).

9. Procédé selon l'une quelconque des revendications précédentes, **caractérisé en ce que** l'on utilise des températures de désorption et des pressions de désorption, qui se situent au-dessus des conditions d'évaporation de l'eau, mais toutefois en dessous des conditions de pyrolyse de l'hémiformal.

10. Procédé selon l'une quelconque des revendications précédentes, **caractérisé en ce que** l'on utilise de l'azote, en tant que gaz inerte, dans la colonne de désorption.

11. Procédé selon l'une quelconque des revendications précédentes, **caractérisé en ce que** la colonne de désorption devient superflue et que l'hémiformal formé dans l'unité d'absorption est introduit directement dans l'unité de pyrolyse.

12. Procédé selon l'une quelconque des revendications précédentes, **caractérisé en ce que** l'on utilise le formaldéhyde, qui a été obtenu par déshydrogénation catalytique du méthanol.
